(19) Europäisches Patentamt
European Patent Office
Office européen des brevets

(11) Veröffentlichungsnummer: **0 084 757**
**A1**

(12) **EUROPÄISCHE PATENTANMELDUNG**

(21) Anmeldenummer: **82810032.1**

(22) Anmeldetag: **25.01.82**

(51) Int. Cl.³: **C 07 D 233/64**, C 07 D 401/04, C 07 D 403/04, C 07 D 405/04, C 07 D 409/04, A 61 K 31/44, A 61 K 31/33

(43) Veröffentlichungstag der Anmeldung: **03.08.83**
**Patentblatt 83/31**

(84) Benannte Vertragsstaaten: **BE DE FR IT LU NL SE**

(71) Anmelder: **CIBA-GEIGY AG, Patentabteilung Postfach, CH-4002 Basel (CH)**

(72) Erfinder: **Sallmann, Alfred, Dr., Joachimsackerstrasse 12, CH-4103 Bottmingen (CH)**

(54) **Trisubstituierte Diaza-Derivate.**

(57) Die Erfindung betrifft neue substituierte Diazaverbindungen, insbesondere Verbindungen der allgemeinen Formel I

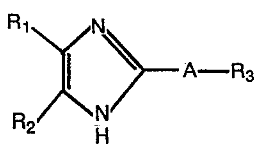

(I),

worin $R_1$ und $R_2$ unabhängig voneinander carbocyclisches Aryl und/oder Heteroaryl bedeuten, A einen zweiwertigen Kohlenwasserstoffrest darstellt und $R_3$ Formyl oder acetalisiertes Formyl bedeutet, ihre Isomeren und ihre Salze, insbesondere pharmazeutisch verwendbare Salze.

Die Verbindungen der Formel (I) können als Hauptphlogistatika verwendet werden und werden nach an sich bekannten Verfahren hergestellt.

EP 0 084 757 A1

CIBA-GEIGY AG
Basel (Schweiz)

4-13773/12985

Trisubstituierte Diaza-Derivate

Die Erfindung betrifft neue substituierte Diazaverbindungen, insbesondere Verbindungen der allgemeinen Formel I

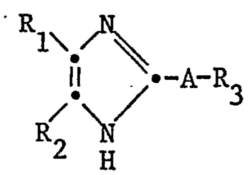

(I),

worin $R_1$ und $R_2$ unabhängig voneinander carbocyclisches Aryl und/oder Heteroaryl bedeuten, A einen zweiwertigen Kohlenwasserstoffrest darstellt und $R_3$ Formyl oder acetalisiertes Formyl bedeutet, ihre Isomeren und ihre Salze, insbesondere pharmazeutisch verwendbare Salze, Verfahren zu ihrer Herstellung, pharmazeutische Präparate, die solche Verbindungen enthalten, und ihre Verwendung, z.B. als Arzneimittelwirkstoffe oder zur Herstellung pharmazeutischer Präparate.

Carbocyclisches Aryl ist beispielsweise monocyclisches carbocyclisches Aryl, wie gegebenenfalls substituiertes Phenyl.

Heteroaryl ist beispielsweise monocyclisches, vorzugsweise 5- oder 6-gliedriges Heteroaryl, wobei mindestens ein Ringglied ein Heteroatom, wie ein Stickstoff-, Sauerstoff- oder Schwefelatom, darstellt, wobei ein Stickstoffatom auch gegebenenfalls in oxidierter Form vorliegen kann. Solche 5-gliedrigen Reste sind z.B. Pyrrolyl, wie 2-Pyrrolyl, Furyl, wie 2-Furyl, Thienyl, wie 2- oder 3-Thienyl.

Als 6-gliedriges Heteroaryl kommt z.B. Pyridyl, wie 2-, 3- oder 4-Pyridyl, 1-Oxidopyridyl, wie 1-Oxido-3-pyridyl oder 1-Oxido-4-pyridyl, und Pyrimidyl, wie 2-Pyrimidyl, in Frage.

Als Substituenten von carbocyclischem Aryl, wie Phenyl, bzw. Heteroaryl, wie Pyridyl oder 1-Oxido-pyridyl, kommen beispielsweise Halogen, Niederalkyl, Hydroxy, Niederalkoxy und/oder Acyloxy in Betracht. Acyloxy ist beispielsweise von einer organischen Carbonsäure abgeleitet und bedeutet z.B. Niederalkanoyloxy.

Ein Kohlenwasserstoffrest A ist beispielsweise ein zweiwertiger aliphatischer, cycloaliphatischer oder cycloaliphatisch-aliphatischer Kohlenwasserstoffrest.

Als zweiwertige aliphatische Kohlenwasserstoffreste kommen beispielsweise Niederalkylen, Niederalkyliden, Niederalkenylen oder Niederalkenyliden in Frage. Zweiwertige cycloaliphatische Kohlenwasserstoffe sind beispielsweise monocyclische 3- bis 8-gliedrige Cycloalkylene oder Cycloalkylidene. Cycloaliphatisch-aliphatische Kohlenwasserstoffreste sind beispielsweise solche, die als cycloaliphatischen Rest einen monocyclischen 3- bis 8-gliedrigen cycloaliphatischen Rest und als aliphatischen Rest Niederalkyliden aufweisen, wie Cycloalkyl-niederalkyliden.

Unter acetalisiertem Formyl ist beispielsweise mit einem aliphatischen Alkohol, wie Niederalkenol, Niederalkendiol oder insbesondere Niederalkanol sowie Niederalkandiol, acetalisiertes Formyl zu verstehen, beispielsweise Dimethoxy-, Methoxy-ethoxy-, Diethoxyformyl oder Methylendioxy-, Ethylendioxy-methyl.

In der vorliegenden Beschreibung sind unter "niederen" organischen Resten und Verbindungen vorzugsweise solche zu verstehen, die bis und mit 7, vor allem bis und mit 4 Kohlenstoffatome (C-Atome) enthalten.

Die vor- und nachstehend verwendeten Allgemeindefinitionen haben im Rahmen der vorliegenden Anmeldung in erster Linie die folgenden Bedeutungen:

Halogen ist z.B. Halogen bis und mit Atomnummer 35, wie Fluor, Chlor oder Brom, ferner Iod.

Niederalkyl ist z.B. Methyl, Ethyl, n-Propyl, Isopropyl, n-Butyl, Isobutyl, sec.-Butyl, tert.-Butyl, ferner ein Pentyl-, Hexyl oder Heptylrest.

Niederalkoxy ist z.B. Methoxy, Ethoxy, n-Propyloxy, Isopropyloxy, n-Butyloxy, Isobutyloxy, sec.-Butyloxy oder tert.-Butyloxy.

Niederalkylthio ist z.B. Methyl-, Ethyl-, n-Propyl-, Isopropyl-, n-Butyl-, Isobutyl-, sec.-Butyl oder tert.-Butylthio.

Phenylniederalkoxy ist z.B. Phenylmethoxy, Phenylethoxy oder Phenylpropyloxy.

Phenylniederalkylthio ist z.B. Benzyl-, Phenylethyl- oder Phenylpropylthio.

Hydroxyniederalkoxy ist z.B. Hydroxyethoxy, Hydroxypropyloxy oder 1,2-Dihydroxypropyloxy.

Niederalkoxyniederalkoxy ist z.B. Methoxyethoxy, Ethoxyethoxy, Methoxypropyloxy oder Methoxybutyloxy.

Phenylniederalkoxyniederalkoxy ist z.B. 2-Benzyloxyethoxy oder 2-(2-Phenylethoxy)-ethoxy.

Niederalkanoyloxy ist z.B. Acetyl-, Propionyl-, Butyryl-, Iso-, sec- oder tert.-Butyryloxy.

Niederalkylen ist z.B. geradkettig, wie Methylen, Ethylen, 1,3-Propylen oder 1,4-Butylen, oder verzweigt, wie 1,2-Propylen, 1,2- oder 1,3-(2-Methyl)-propylen oder 1,2-Butylen.

Niederalkyliden weist ein tertiäres oder insbesondere ein quartäres C-Atom auf und ist z.B. Ethyliden oder 1,1- oder 2,2-Propyliden, ferner 1,1- oder 2,2-Butyliden oder 1,1-, 2,2- oder 3,3-Pentyliden.

Niederalkenylen ist z.B. Ethenylen, 1,2- oder 1,3-Propenylen oder 1,2-, 1,3- oder 1,4-Buten-2-ylen.

Niederalkenyliden ist z.B. Ethenyliden, 1,1-Propen-1-yliden, 1,1-Propen-2-yliden, ferner ein Butenyliden, wie 1,1-Buten-3-yliden.

Cycloalkylen ist z.B. Cyclopropylen, 1,2- oder 1,3-Cyclobutylen, 1,2-, 1,3- oder 1,4-Cyclopentylen, ferner ein Cyclohexylen.

Cycloalkyliden ist z.B. Cyclopropyliden, Cyclobutyliden, Cyclopentyliden oder Cyclohexyliden.

Cycloalkyl-niederalkyliden ist z.B. ein Cyclopropyl-, Cyclobutyl-, Cyclopentyl- oder Cyclohexyl-methylen , -ethyliden oder -propyliden, ferner ein Cyclohexyl-butyliden.

Carboxyniederalkoxy ist z.B. Carboxymethoxy, 2-Carboxyethoxy, 2-, 3-Carboxypropyloxy, 1-Carboxy-2-propyloxy, 2-, 3- oder 4-Carboxy-n-butyloxy, 1-Carboxy-2-methyl-propyl-3-oxy oder 1-Carboxy-2-methyl-propyl-2-oxy.

Niederalkoxycarbonyl-niederalkoxy enthält jeweils im Niederalkoxyteil unabhängig voneinander die vorstehend unter Niederalkoxy aufgeführten Bedeutungen.

Salze von erfindungsgemässen Verbindungen der Formel I sind vorzugsweise pharmazeutisch verwendbare Salze, wie pharmazeutisch verwendbare Säureadditionssalze, beispielsweise Salze mit anorganischen Säuren, wie Mineralsäure, mit Sulfaminsäuren, wie Cyclohexylsulfaminsäure, mit organischen Carbonsäuren, wie Niederalkancarbonsäuren, gegebenenfalls ungesättigte Dicarbonsäuren, mit durch Hydroxy und/oder Oxo substituierten Carbonsäuren oder mit Sulfonsäuren, beispielsweise Sulfate oder Hydrohalogenide, wie Hydrobromide oder Hydrochloride, Oxalate, Malonate, Fumarate oder Maleinate, Tartrate, Pyruvate oder Citrate, Sulfonate, wie Methan-, Benzol- oder p-Toluolsulfonate.

Die Verbindungen der Formel I und ihre pharmazeutisch verwendbaren Salze weisen wertvolle pharmakologische Eigenschaften auf. Insbesondere besitzen sie, z.B. bei lokaler Anwendung, eine ausgeprägte antiinflammatorische Wirkung.

Diese Eigenschaft lässt sich beispielsweise nach der von G. Tonelli, L. Thibault, Endocrinology 77, 625 (1965) entwickelten Methode durch Hemmung des mit Crotonöl induzierten Rattenohroedems bei der Normalratte im Dosisbereich von etwa 1 bis etwa 100 mg/ml nachweisen. So wurde beispielsweise für die Verbindung 2-[4(5)-Phenyl-5(4)-(3-pyridyl)-imidazol-2-yl]-acetaldehyd-dimethylacetal im vorstehend beschriebenen Test ein $ED_{50}$-Wert von 18 mg/ml ermittelt.

Die erfindungsgemäss anwendbaren Verbindungen der Formel I sind daher als Arzneimittel, insbesondere externe (topische) Hautphlogistatika für die Behandlung entzündlicher Dermatosen jeglicher Genese, wie bei leichten Hautirritationen, Kontaktdermatitis, Exanthemen, Verbrennungen, sowie als Schleimhautphlogostatika für die Behandlung von Mukosaentzündungen, z.B. der Augen, Nase, Lippen, Mund, Genital-, Analregion, geeignet. Ferner können die Verbindungen als Sonnenschutzmittel verwendet werden.

Die vorliegende Erfindung betrifft ebenfalls die Verwendung der erfindungsgemässen Verbindungen und deren Salze zur Behandlung von Entzündungen, z.B. von entzündlichen Erkrankungen unterschiedlichster Genese, sowie zur Herstellung von Arzneimitteln.

Die Erfindung betrifft insbesondere Verbindungen der Formel I, worin einerseits $R_1$ und $R_2$ unabhängig voneinander Phenyl und/oder durch Halogen, Niederalkyl, Hydroxy, Niederalkoxy und/oder Niederalkanoyl substituiertes Phenyl bedeuten oder worin andererseits einer der Reste $R_1$ und $R_2$ Pyrrolyl, Furyl, Thienyl, Pyridyl, 1-Oxidopyridyl oder Pyrimidyl bedeutet, welches jeweils unsubstituiert oder durch Halogen, Niederalkyl, Hydroxy, Niederalkoxy und/oder Niederalkanoyloxy substituiert sein können, und der andere Phenyl, Pyrrolyl, Furyl, Thienyl, Pyridyl, 1-Oxidopyridyl oder Pyrimidyl bedeutet, welches jeweils unsubstituiert oder durch Halogen, Niederalkyl, Hydroxy, Niederalkoxy

und/oder Niederalkanoyloxy substituiert sein können, und jeweils A Niederalkylen, Niederalkyliden, Niederalkenylen, Niederalkenyliden, Cycloalkylen, Cycloalkyliden oder Cycloalkyl-niederalkyliden darstellen und $R_3$ Formyl oder mit einem aliphatischen Alkohol acetalisiertes Formyl bedeutet, ihre Isomeren sowie ihre Salze, insbesondere pharmazeutisch verwendbare Salze.

Die Erfindung betrifft insbesondere Verbindungen der Formel I, worin $R_1$ und $R_2$ unabhängig voneinander Phenyl und/oder durch Halogen, Hydroxy, Niederalkyl, Niederalkoxy und/oder Niederalkanoyloxy substituiertes Phenyl bedeuten, A Niederalkylen mit bis und mit 4 C-Atomen, wie Methylen, Niederalkyliden mit bis und mit 7 C-Atomen, wie 2,2-Propyliden, Niederalkenylen mit bis und mit 4 C-Atomen, wie 1,3-Propen-2-ylen, Niederalkenyliden mit bis und mit 7 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkyl-niederalkyliden mit bis und mit 7 C-Atomen im Alkylidenteil und mit einem 3- bis 8-gliedrigem Cycloalkylteil, wie 2-Cyclohexyl-1,1-ethyliden, bedeutet und $R_3$ Formyl oder mit einem Niederalkanol, Niederalkenol, Niederalkandiol oder Niederalkendiol acetalisiertes Formyl bedeutet, ihre Isomeren sowie ihre Salze, insbesondere pharmazeutisch verwendbare Salze.

Die Erfindung betrifft insbesondere Verbindungen der Formel I, worin einer der Reste $R_1$ und $R_2$ Pyridyl oder 1-Oxido-pyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, wie Methylen, Niederalkyliden mit bis und mit 7 C-Atomen, wie 2,2-Propyliden, Niederalkenylen mit bis und mit 7 C-Atomen, wie 1,3-Propen-2-ylen, Niederalkenyliden mit bis und mit 4 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkyl-niederalkyliden mit bis und mit 7 C-Atomen im Alkylidenteil und mit einem 3- bis 8-gliedrigem Cycloalkylteil, wie 2-Cyclohexyl-1,1-ethyliden, bedeutet und $R_3$ mit einem Niederalkanol, Niederalkenol, Niederalkandiol oder Niederalkendiol acetalisiertes Formyl bedeutet, ihre Isomeren sowie ihre Salze, insbesondere pharmazeutisch verwendbare Salze.

Die Erfindung betrifft insbesondere Verbindungen der Formel I, worin $R_1$ und $R_2$ unabhängig voneinander Phenyl und/oder durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeuten, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, darstellen und $R_3$ Formyl oder Diniederalkoxy-methyl jeweils mit bis und mit 4 C-Atomen im Niederalkoxyteil, wie Dimethoxy-methyl, oder Niederalkendioxy-methyl mit bis und mit 4 C-Atomen im Niederalkylenteil, wie 1,3-Dioxolan-2-yl, bedeutet, ihre Isomeren sowie ihre Salze, insbesondere pharmazeutisch verwendbare Salze.

Die Erfindung betrifft insbesondere Verbindungen der Formel I, worin einer der Reste $R_1$ und $R_2$ Phenyl oder durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeutet und der andere Pyridyl, wie 3-Pyridyl, oder 1-Oxido-pyridyl, wie 1-Oxido-3-pyridyl, bedeutet, die jeweils unsubstituiert oder durch Halogen mit Atomnummer bis und mit 35, wie Chlor, Hydroxy und/oder Niederalkoxy mit bis und mit 4 C-Atomen, wie Methoxy, substituiert sein können, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, dartellen und $R_3$ Formyl oder Diniederalkoxy-methyl jeweils mit bis und mit 4 C-Atomen im Niederalkylteil, wie Dimethoxymethyl, oder Niederalkyendioxy-methyl mit bis und mit 4 C-Atomen im Niederalkylenteil, wie 1,3-Dioxolan-2-yl, bedeutet, ihre Isomeren sowie ihre Salze, insbesondere pharmazeutisch verwendbare Salze.

Die Erfindung betrifft insbesondere Verbindungen der Formel I, worin $R_1$ und $R_2$ unabhängig voneinander Phenyl und/oder durch Niederalkoxy mit bis und mit 4 C-Atomen, wie Methoxy, substituiertes Phenyl bedeuten, A Niederalkylen mit bis und mit 4 C-Atomen, wie Methylen, oder insbesondere Niederalkyliden mit bis und mit 4 C-Atomen, wie 2,2-Propyliden, darstellt und $R_3$ Formyl oder Diniederalkoxy-methyl jeweils mit bis und mit 4 C-Atomen im Niederalkoxyteil, wie Diethoxy-methyl, oder Niederalkylendioxy-methyl mit bis und mit 4 C-Atomen im Niederalkylenteil, wie 1,3-Dioxolan-2-yl, bedeutet, ihre Isomeren sowie ihre Salze, insbesondere pharmazeutisch verwendbare Salze.

Die Erfindung betrifft in erster Linie Verbindungen der Formel I, worin einer der Reste $R_1$ und $R_2$ Phenyl oder durch Halogen mit Atomnummer bis und mit 35, wie Chlor, Hydroxy oder Niederalkoxy mit bis und mit 4 C-Atomen, wie Methoxy, substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A Niederalkyliden mit bis und mit 4 C-Atomen, wie 2,2-Propyliden, bedeutet, und $R_3$ Formyl oder Diniederalkoxy-methyl jeweils mit bis und mit 4 C-Atomen im Niederalkoxyteil, wie Diethoxy-methyl, oder Niederalkylendioxy-methyl mit bis und mit 4 C-Atomen im Niederalkylenteil, wie 1,3-Dioxolan-2-yl, bedeutet, ihre Isomeren sowie ihre Salze, insbesondere pharmazeutisch verwendbare Salze.

Die Erfindung betrifft in erster Linie Verbindungen der Formel I, worin einer der Reste $R_1$ und $R_2$ Phenyl oder durch Halogen mit Atomnummer bis und mit 35, wie Chlor, Hydroxy oder Niederalkoxy mit bis und mit 4 C-Atomen, wie Methoxy substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A ein ein quartäres C-Atom aufweisendes Niederalkyliden mit bis und mit 4 C-Atomen, wie 2,2-Propyliden, wobei das quartäre C-Atom direkt an den Imidazolring gebunden ist, bedeutet, und $R_3$ Formyl oder Diniederalkoxy-methyl mit bis und mit 4 C-Atomen jeweils im Niederalkylteil, wie Diethoxy-methyl, darstellt, ihre Isomeren sowie ihre Salze, insbesondere pharmazeutisch verwendbare Salze.

Die Erfindung betrifft in allererster Linie Verbindungen der Formel I, worin einer der Reste $R_1$ und $R_2$ Phenyl und der andere Pyridyl, wie 3-Pyridyl, oder 1-Oxidopyridyl, wie 1-Oxido-3-pyridyl, bedeutet, A 2,2-Propyliden darstellt und $R_3$ Formyl oder Diniederalkoxy-methyl jeweils mit bis und mit 4 C-Atomen im Niederalkyl- wie Diethoxy-methyl, bedeutet, ihre Isomeren sowie ihre Salze, insbesondere pharmazeutisch verwendbare Salze.

Die Erfindung betrifft namentlich die in den Beispielen genannten neuen Verbindungen und ihre Salze, insbesondere pharmazeutisch verwendbare Salze von solchen Verbindungen mit salzbildenden Gruppen, sowie die in den Beispielen aufgeführten topisch applizierbaren pharmazeutischen Präparate.

Die Erfindung betrifft ebenso Verfahren zur Herstellung von Verbindungen der Formel I und ihrer Salze, insbesondere pharmazeutisch verwendbarer Salze von solchen Verbindungen mit salzbildenden Gruppen, sowie die in den Beispielen aufgeführten Herstellungsverfahren.

Die Verbindungen der Formel I oder deren Salze lassen sich in an sich bekannter Weise herstellen.

Eine Verfahrensweise besteht beispielsweise darin, dass man aus einer Verbindung der Formel

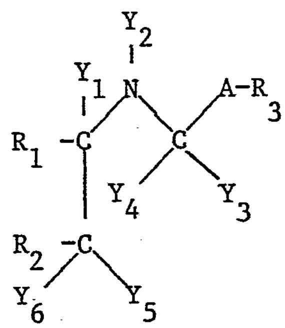

(II) ,

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N- bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel -NH- darstellt oder worin $Y_1$ gemeinsam mit $Y_6$ eine Bindung darstellt, $Y_2$ gemeinsam mit $Y_3$ eine zusätzliche Bindung und einer der Reste $Y_4$ und $Y_5$ Amino und der andere Amino, Hydroxy oder reaktionsfähiges verestertes Hydroxy, insbesondere Halogen oder Sulfonyloxy, darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo oder Imino bedeutet, oder einem Tautomeren und/oder Salz davon, unter Einführung einer gegebenenfalls zusätzlichen Bindung H-Z abspaltet, und gewünschtenfalls die verfahrensgemäss erhältliche Verbindung in eine andere Verbindung der Formel I, eine verfahrensgemäss erhältliche freie Verbindung in ein Salz oder ein verfahrensgemäss erhältliches Salz in die freie Verbindung oder in ein anderes Salz überführt und/oder, wenn erwünscht, ein erfindungsgemäss erhältliches Gemisch von isomeren Verbindungen der Formel I in die einzelnen Isomeren auftrennt.

Dabei bedeutet Z Hydroxy oder Amino $Y_1$ bzw. $Y_6$, $Y_3$ oder $Y_4$ bzw. $Y_5$ oder Halogen bzw. Sulfonyloxy $Y_4$ bzw. $Y_5$.

Reaktionsfähiges verestertes Hydroxy ist beispielsweise mit einer anorganischen Mineralsäure, wie Halogenwasserstoffsäure, oder mit einer organischen Sulfonsäure, wie Niederalkan- oder gegebenenfalls

substituierte Benzolsulfonsäure, verestertes Hydroxy und bedeutet in erster Linie Halogen, z.B. Chlor oder Brom, sowie Sulfonyloxy, z.B. Methan- oder p-Toluolsulfonyloxy.

Tautomere von Verbindungen der Formel II sind beispielsweise solche, in denen eine partielle Enol- bzw. Enamin-Gruppierung der Formel

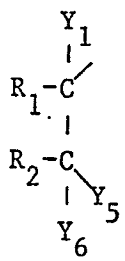

(IIa) ,

worin $Y_1$ gemeinsam mit $Y_6$ eine zusätzliche Bindung darstellt und $Y_5$ Hydroxy bzw. Amino bedeutet, in Form der entsprechenden tautomeren Keto- bzw. Ketiminform, worin $Y_1$ Wasserstoff ist und $Y_5$ gemeinsam mit $Y_6$ Oxo bzw. Imino bedeutet, vorliegt und/oder solche, in denen eine partielle Enol- bzw. Enamingruppierung der Formel

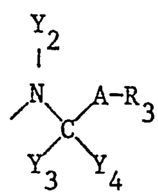

(IIb) ,

worin $Y_2$ gemeinsam mit $Y_3$ eine Bindung darstellt und $Y_4$ Hydroxy bzw. Amino bedeutet, in Form der entsprechenden tautomeren Form vorliegt, worin $Y_2$ Wasserstoff ist und $Y_3$ gemeinsam mit $Y_4$ Oxo bzw. Imino darstellt, vorliegt, wobei die genannten Tautomeren miteinander im Gleichgewicht stehen.

Die Abspaltung von H - Z aus einer Verbindung der Formel II, einem Tautomeren und/oder Salz davon erfolgt in üblicher, insbesondere in der aus der Literatur für analoge Reaktionen bekannten Weise, erforderlichenfalls unter Erwärmen, wie in einem Temperaturbereich von etwa 20° bis etwa 250°C, unter Druck und/oder in Gegenwart eines katalytischen Mittels, vorzugsweise einer Säure. Als Säuren eignen sich

beispielsweise anorganische Säuren, wie Mineralsäuren, z.B. Schwefelsäure, Polyphosphorsäure oder Halogenwasserstoffsäure, wie Chlorwasserstoffsäure, oder organische Säuren, wie Niederalkancarbonsäuren, z.B. Essigsäure. Dabei arbeitet man erforderlichenfalls in einem inerten Lösungsmittel, beispielswisse einem gegebenenfalls halogenierten Kohlenwasserstoff, wie Chloroform, Chlorbenzol, Hexan oder Toluol, einem Niederalkanol, wie Methanol oder Ethanol, einem Carbonsäureamid, wie Niederalkancarbonsäureamid, z.B. Dimethylformamid oder Formamid, oder einer Niederalkancarbonsäure, wie Ameisen- oder Essigsäure, und/oder unter Inertgas, wie Stickstoff.

Die Ausgangsstoffe der Formel II, ihre Tautomeren und/oder Salze werden nach an sich bekannten Verfahren zum überwiegenden Teil in situ gebildet und unter den Reaktionsbedingungen ohne Isolierung weiter zu der Verbindung der Formel I umgesetzt. Dabei kann die Abspaltung von H - Z unter direkter Cyclisierung oder im Anschluss an eine vorgelagerte Cyclisierung erfolgen.

So kann man in einer bevorzugten Ausführungsform des vorstehenden Verfahren beispielsweise ein acyliertes α-Aminoketon der Formel

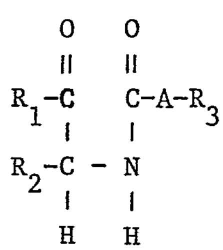

(IIIa)

oder ein Salz davon mit Ammoniak umsetzen. Dabei arbeitet man beispielsweise unter Erwärmen, z.B. in einem Temperaturbereich von etwa 50° bis etwa 250°C, und unter inerten Bedingungen.

Die Ausgangsstoffe der Formel (IIIa) sind bekannt oder werden nach an sich bekannten Verfahren hergestellt. Beispielsweise geht man von einer Verbindung der Formel

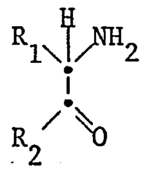

(IIIb)

oder einem Salz davon aus und setzt diese mit einem Säurederivat der Formel $R_3$-A-COOH (IIIc), beispielsweise einem entsprechenden Anhydrid, wie einer Halogencarbonylverbindung, um.

In einer weiteren, besonders bevorzugten Variante des eingangs beschriebenen Verfahrens setzt man eine Verbindung der Formel

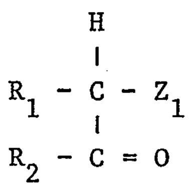

(IIId)

worin $Z_1$ gegebenenfalls reaktionsfähiges verestertes Hydroxy bedeutet, oder ein Salz davon mit einer Verbindung der Formel

$R_3 - A - Z_2$ (IIIe),

worin $Z_2$ ein Amidinorest oder Ammoniumcarboxylat bedeutet, oder ein Salz davon gegebenenfalls mit Ammoniak um.

Die Umsetzung mit einem Amidin der Formel (IIIe) erfolgt üblicherweise unter Erwärmen, beispielsweise in einem Temperaturbereich von etwa 50° bis etwa 250°C.

Die Reaktion des Ammoniumcarboxylats der Formel (IIIe) mit einer Verbindung der Formel (IIId) wird mit einem mindestens 3-molaren, oder, falls die Verbindung der Formel (IIId) in Salzform vorliegt, mindestens 4-molaren Ueberschuss des Ammoniumsalzes der Verbindung der Formel (IIIe), gegebenenfalls unter Erwärmen, z.B. in einem Temperaturbereich von etwa 50° bis etwa 250°C, vorzugsweise bei 90° bis 120°C, durchgeführt, wobei die Verbindung der Formel (IIIe) gleichzeitig als

Lösungsmittel dienen kann. Diese Variante kann beispielsweise auch dahingehend abgewandelt werden, dass man das Ammoniumsalz der Formel IIIe, in bezug auf den reaktionsfähigen Ester $Z_1$, in etwa äquimolarer Menge einsetzt und zusätzlich Ammoniak, gegebenenfalls in Form eines Salzes einer gegenüber $R_3$-COOH schwächeren Säure, in überschüssiger Menge, vorzugsweise in 3- bis 5-fachem Ueberschuss, zugibt.

Eine reaktionsfähige veresterte Hydroxygruppe $Z_1$ ist beispielsweise eine vorzugsweise durch starke anorganische oder organische Säuren, wie starke Mineralsäuren, z.B. Halogenwasserstoffsäuren, wie Chlor- oder Bromwasserstoffsäure, oder starke organische Sulfonsäuren, wie entsprechende Niederalkan- oder Arylsulfonsäuren, z.B. Methan- oder eine gegebenenfalls substituierte Benzolsulfonsäure, veresterte Hydroxygruppe und stellt z.B. Halogen, wie Chlor oder Brom, Niederalkylsulfonyloxy, z.B. Methyl- oder Ethylsulfonyloxy, oder Arylsulfonyloxy, z.B. p-Toluol- oder Benzolsulfonyloxy, dar.

Das Ammoniumsalz der Formel (IIIe) kann auch unter den Reaktionsbedingungen in situ gebildet werden, beispielsweise indem man im Reaktionsgemisch die freie Säure der Formel (IIIe) vorlegt und mit flüssigem oder gasförmigem Ammoniak versetzt. Bei dieser Ausführungsform kann das Ammoniak auch in Form eines Salzes mit einer gegenüber $R_3$-A-COOH schwächeren Säure, wie Kohlensäure, zugegeben werden.
Als geeignete Lösungsmittel kommen z.B. gegebenenfalls halogenierte Kohlenwasserstoffe, wie gegebenenfalls halogenierte aliphatische, cycloaliphatische oder aromatische Kohlenwasserstoffe, wie Hexan, Cyclohexan, Toluol, Chloroform, oder Chlorbenzol, Alkanole, wie Propanol, Isopropanol, Butanole, Pentanole oder Octanole, Ether, wie Dimethoxyethan, Ethylenglykolmonoethylether, Dioxan oder Tetrahydrofuran, Niederalkancarbonsäuren, wie Ameisen- oder Essigsäure oder vorzugsweise Säuren der Formel (IIIc), Amide, wie Niederalkancarbonsäureamide, z.B. Formamid oder Dimethylformamid, sowie Lactame, z.B. N-Methylpyrrolidon, Sulfoxide, wie Dimethylsulfoxid, oder Wasser in Frage.

Eine bevorzugte Ausführung dieser Variante zur erfindungsgemässen Darstellung von Verbindungen der Formel I über Verbindungen der Formel II besteht darin, dass man eine Verbindung der Formel IIId, worin $Z_1$ beispielsweise Halogen, wie Brom, bedeutet, mit einem Ammoniumsalz der Formel (IIIe) bei einer Reaktionstemperatur von etwa 100°C umsetzt. Die Verbindung der Formel (IIIe) wird im Ueberschuss zugegeben, beispielsweise in einem Verhältnis gegenüber dem Ester der Formel (IIId) von etwa 4:1 bis etwa 6:1, und lässt sich in situ bilden, indem man z.B. die entsprechende Säure unter den Reaktionsbedingungen mit flüssigem Ammoniak umsetzt.

Die Ausgangsstoffe der Formel (IIId) sind bekannt oder können nach an sich bekannten Verfahren hergestellt werden.

So lassen sie sich beispielsweise durch Esterkondensation von veresterten Säuren der Formeln $R_1$-$CH_2$-COOH bzw. $R_2$-$CH_2$-COOH mit veresterten Säuren der Formeln $R_1$-COOH bzw. $R_2$-COOH, vorzugsweise in Gegenwart einer Base erhalten. Das resultierende α-Methylenketon der Formel

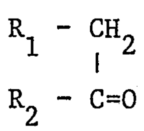

(IIIf)

wird beispielsweise bromiert und somit in eine Verbindung der Formel (IIId) bzw. ein Salz, z.B. Hydrohalogenid, davon überführt, worin $Z_1$ Brom bedeutet.

Ausserdem kann man in einer weiteren bevorzugten Ausführungsform ein Oxazol der Formel

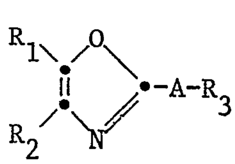

(IIIg)

mit Ammoniak über Verbindungen der Formel II zu Verbindungen der Formel I umsetzen.

Diese Reaktion wird gegebenenfalls unter Druck, beispielsweise bei 185 atü, und/oder Erwärmen, z.B. auf etwa 100° bis etwa 250°C, durchgeführt.

Die Verbindungen der Formel (IIIg) ihrerseits lassen sich nach an sich bekanntem Verfahren herstellen, beispielsweise durch Umsetzung von Verbindungen der Formel

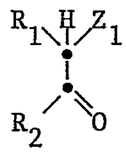 (IIIh),

worin $Z_1$ gegebenenfalls reaktionsfähiges verestertes Hydroxy bedeutet, mit einer Carbonsäure der Formel $R_3$-A-COOH (IIIc), einem funktionellen Derivat oder Salz davon, wie einem entsprechenden Anhydrid, z.B. einem Halogencarbonylderivat, gegebenenfalls über eine Verbindung der Formel

$$R_2\text{-C(=O)-CH}(R_1)\text{-O-C(=O)-A-}R_3 \qquad \text{(IIIi)},$$

und mit Ammoniak.

Dabei wird jeweils eine Verbindung der Formel II gebildet, die erfindungsgemäss, insbesondere in situ, zu einer Verbindung der Formel I weiterreagiert.

In einer weiteren bevorzugten Ausführungsform des vorstehenden Verfahrens kann man beispielsweise das Diketon der Formel

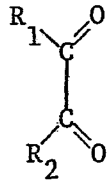 (IIIh)

mit einem Aldehyd der Formel $R_3$-A-C(=O)-H (IIIl) oder einem Salz davon, und mit einem Ueberschuss an Ammoniak unter Erwärmen umsetzen. Dabei wird beispielsweise intermediär eine Verbindung der Formel (II), z.B. eine solche, worin $Y_1$ Hydroxy, $Y_2$ sowie $Y_6$ Wasserstoff bedeuten und $Y_3$ gemeinsam mit $Y_4$ und $Y_5$ eine Gruppe der Formel =N- darstellt, z.B.

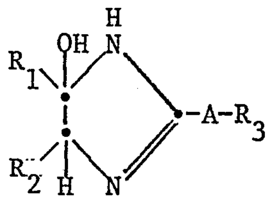 (IIIm) oder eine tautomere Form

davon gebildet, die unter den Reaktionsbedingungen erfindungsgemäss weiterreagiert.

Dabei wird jeweils eine Verbindung der Formel II gebildet, die erfindungsgemäss, insbesondere in situ, zu einer Verbindung der Formel I weiterreagiert.

Einige der vorstehend beschriebenen Verfahrensvarianten lassen sich durch Anwendung milder Bedingungen so durchführen, dass die Verbindungen der Formel II bzw. ihre Tautomeren und/oder Salze isoliert werden können.

Verbindungen der Formel I oder Salze davon kann man weiterhin herstellen, indem man beispielsweise eine Verbindung der Formel

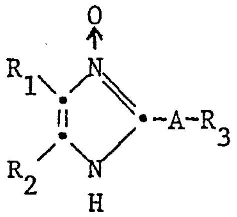 (IV)

oder ein Salz davon zu einer Verbindung der Formel I reduziert und gewünschtenfalls die verfahrensgemäss erhältliche freie Verbindung in ein Salz oder ein verfahrensgemäss erhältliches Salz in die freie Verbindung oder in ein anderes Salz überführt.

Die Reduktion erfolgt nach an sich bekannten Verfahren. So behandelt man Verbindungen der Formel (IV) oder deren Salze mit Wasserstoff in Gegenwart eines Hydrierungskatalysators oder mit einem Dithionit, z.B. Natriumdithionit, oder mit einem Phosphorhalogenid, z.B. Phosphortrichlorid. Als Hydrierungskatalysatoren lassen sich beispielsweise Elemente der VIII. Nebengruppe sowie Derivate davon, wie Platin, Palladium oder Palladiumchlorid, die gegebenenfalls auf einem üblichen Trägermaterial, wie Aktivkohle oder Erdalkalimetallverbindungen, z.B. Bariumcarbonat, aufgezogen sind, oder Raney-Nickel, verwenden.

Die Reduktion lässt sich erforderlichenfalls unter Kühlen oder Erwärmen, beispielsweise in einem Temperaturbereich von etwa 0° bis etwa 150°C, in einem inerten Lösungsmittel, wie einem halogenierten Kohlenwasserstoff, z.B. Chloroform, Tetrachlorkohlenstoff oder Chlorbenzol, oder einem Ether, wie Dimethoxyethan, Diethylether, Dioxan oder Tetrahydrofuran, und/oder unter Inertgas, z.B. Stickstoff, durchführen.

Das vorstehend beschriebene Herstellungsverfahren wird in erster Linie mit solchen Verbindungen der Formel (IV) durchgeführt, worin $R_3$ acetalisiertes Formyl darstellt.

Die Ausgangsstoffe der Formel IV oder deren Salze sind in an sich bekannter Weise erhältlich, beispielsweise in dem eine Verbindung der Formel

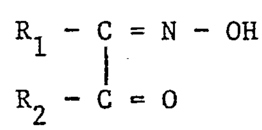

(IVa),

ein Tautomeres oder ein Salz davon <u>in situ</u> mit einem Ueberschuss an Ammoniak und einem Aldehyd der Formel $R_3$-A-C(=O)-H (III1) bei erhöhter Temperatur umsetzt.

Verbindungen der Formel I lassen sich ferner herstellen, indem man in einer Verbindung der Formel

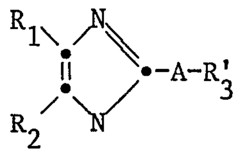
(V),

worin $R_3'$ einen im $R_3$ überführbaren Rest bedeutet, den Rest $R_3'$ in einen Rest $R_3$ überführt und gewünschtenfalls die verfahrensgemäss erhältliche freie Verbindung in ein Salz oder ein verfahrensgemäss erhältliches Salz in die freie Verbindung oder in ein anderes Salz überführt.

Derartige Gruppen $R_3'$ sind beispielsweise reduktiv in den Rest $R_3$ überführbare Gruppen, wie gegebenenfalls funktionell abgewandeltes Carboxy. Insbesondere werden Carboxy, Halogencarbonyl, Niederalkoxycarbonyl oder Carbamoyl $R_3'$ durch Reduktion in Formyl $R_3$ überführt.

Die Reduktion der genannten Reste $R_3'$ erfolgt in an sich bekannter Weise, beispielsweise unter Verwendung eines geeigneten Reduktionsmittels in einem inerten Lösungsmittel, gegebenenfalls unter Kühlen oder Erwärmen. So wird beispielsweise Halogencarbonyl durch Wasserstoff an Edelmetallkatalysatoren, wie Palladium, welche gegebenenfalls an Trägermaterialien, wie Bariumsulfat, gebunden sind, zu Formyl reduziert, während Reduktion von Carboxy zu Formyl beispielsweise mit Hilfe von Ameisensäure oder gegebenenfalls komplexen Hydriden, wie Lithiumaluminiumhydrid oder Lithyium-tri-tert.-butoxy- oder Lithium-triethoxy-aluminat, reduziert wird. Ebenso kann Niederalkoxycarbonyl bzw. Carbamoyl mit Hilfe von geeigneten gegebenenfalls komplexen Hydriden reduktiv zu Formyl reduziert werden.

Weitere in $R_3$ überführbare Gruppen sind beispielsweise oxidativ in diesen überführbare Reste, wie gegebenenfalls verestertes oder veräthertes Hydroxymethyl.

Verestertes Hydroxymethyl ist beispielsweise mit einer Mineralsäure, wie Halogenwasserstoff-, wie Chlorwasserstoffsäure, oder einer Carbonsäure, wie Niederalkancarbonsäure, z.B. Essigsäure, oder gegebenenfalls substituierten Benzoesäure verestertes Hydroxymethyl. Verethertes Hydroxymethyl ist beispielsweise mit einem Niederalkanol verethert.

Die Oxidation derartiger Gruppen $R_3'$ erfolgt in an sich bekannter Weise, beispielsweise durch Umsetzung in einem geeigneten Oxidationsmittel, beispielsweise in einem inerten Lösungsmittel, wie einer Niederalkylcarbonsäure z.B. Essigsäure, einem Keton, z.B. Aceton, einem Ether, z.B. Tetrahydrofuran, einem heterocyclischen Aromaten, z.B. Pyridin, oder Wasser oder einem Gemisch davon, erforderlichenfalls unter Kühlen oder Erwärmen, z.B. von etwa 0° bis etwa 150°C. Als Oxidationsmittel kommen beispielsweise oxidierende Uebergangsmetallverbindungen, insbesondere solche mit Elementen der I., VI., VII., oder VIII. Nebengruppe, in Frage. Als Beispiele seien genannt: Silberverbindungen, wie Silbernitrat, -oxid oder picolinat, Chromverbindungne, wie Chromtrioxid oder Kaliumdichromat, Manganverbindungen, wie Tetrabutylammonium- oder Benzyl(triethyl)-ammoniumpermanganat, ferner Eisenverbindungen, wie Kaliumferrat. Insbesondere werden selektive Oxidationsmittel verwendet, wie Pyridiniumchlorochromat, ein geeignetes Keton, wie Cyclohexanon in Gegenwart von Aluminium-tert.-butylat, oder, wenn Hydroxymethyl mit p-Toluolsulfonsäure reaktionsfähig verestert ist, Dimethylsulfoxid.

So wird beispielsweise Hydroxymethyl $R_3'$ mit Bis-tetrabutylammoniumdichromat, während z.B. mit Chlorwasserstoffsäure verestertes Hydroxymethyl $R_3'$ mit 4-Dimethylamino-pyridin-N-oxid zu Formyl $R_3$ oxidiert wird.

Weitere, in $R_3$ überführbare Gruppe $R_3'$ sind funktionell abgewandelte Formylgruppen oder durch Schutzgruppen geschütztes Formyl. Funktionell

abgewandeltes Formyl weist als funktionell abgewandeltes Oxo beispielsweise Thioxo, gegebenenfalls N-substituiertes Imino, wie N-Niederalkyl- oder N-Phenyl-imino, Hydroxyimino, gegebenenfalls substituiertes Hydrazono, wie N-Tosyl- oder N,N-Diniederalkyl-hydrazono, auf. Derartig funktionell abgewandeltes Formyl wird in üblicher Weise hydrolytisch, erforderlichenfalls in Gegenwart einer Protonsäure, in Formyl $R_3$ übergeführt. Durch Schutzgruppen geschütztes Formyl ist beispielsweise mit einem Mercaptan, wie Niederalkandiol oder Niederalkylendiol, oder mit einem Mercaptan und Alkohol, wie Niederalkanol und Niederalkanthiol oder Mercaptoniederalkanol, acetalisiertes Formyl. Weiterhin kann Formyl als Di- oder Tetrahydro-1,3-oxazin, wie 3,3,5-Trimethyl-1,3-oxazin-2-yl, oder als Imidazolidin, wie gegebenenfalls N-mono- oder N,N-disubstituiertes Imidazolidin-2-yl, sowie als Halbacetal mit einem Alkohol, wie Niederalkanol, oder mit einem Mercaptan, wie Niederalkanthiol, vorliegen. Entsprechende Schutzgruppen können oxidativ, hydrolytisch, erforderlichenfalls in Gegenwart einer Protonsäure abgespaltet werden. Insbesondere werden entsprechende Thioacetale, bzw. -halbacetale oxidativ, beispielsweise unter Einwirkung von N-Bromsuccinimid, Chloramin-T, Thallium-(III)-nitrit, Schwermetallverbindungen, z.B. Quecksilber(II)oxid, Kupfer(II)oxidbarchlorid, oder elektrochemisch in Formyl übergeführt werden, während aus entsprechenden Oxazin- bzw. Imidazolidin-Derivaten sowie Halbacetalen mit Niederalkanolen der Rest Formyl hydrolytisch in Gegenwart von starken Protonsäure, wie Mineralsäuren, z.B. Schwefelsäure, Halogenwasserstoffsäuren oder Phosphorsäuren, wie Sulfonsäuren,z.B. p-Toluolsulfonsäure, oder wie Carbonsäuren, z.B. Eisessig, freigesetzt werden.

Die Ausgangsstoffe der Formel V werden nach an sich bekannten Verfahren hergestellt. Beispielsweise geht man von einem Diketon der Formel

$$\begin{array}{l} R_1 - C = O \\ \qquad | \\ R_2 - C = O \end{array} \qquad \text{(IIIk)}$$

aus und setzt dieses mit Ammoniak und einem Aldehyd der Formel $R_3'$-A-C(=O)-H in einem inerten Lösungsmittel und unter Erwärmen in

die in situ gebildete Verbindung der Formel V ohne Isolierung weiter um.

Der Aldehyd der Formel $R_3$-A-C(=O)-H (III1) kann in den vorstehend beschriebenen Herstellungsverfahren für Verbindungen der Formeln II und IV beispielsweise auch unter den Reaktionsbedingungen aus einem Oxazinderivat der Formel

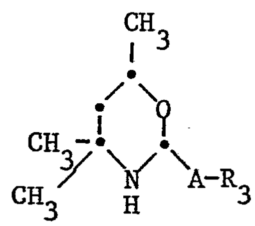

(V)

freigesetzt werden. Die Verbindungen der Formel (V) lassen sich beispielsweise herstellen, indem man 2-Methyl-2,4-pentandiol mit einem Nitril der Formel $R_3$-A-CN in Gegenwart von Schwefelsäure umsetzt. Das dabei gebildete, entsprechend substituierte Dihydro-1,3-oxazin wird in einem Gemisch aus Tetrahydrofuran und Ethanol bei -45°C und einem pH-Wert von etwa 7 unter Einwirkung von Natriumborhydrid zu dem Tetrahydro-1,3-oxazin der Formel V reduziert.

In den vorstehend beschriebenen Verfahren zur Herstellung von Verbindungen der Formeln II, IV und V kann das Ammoniak, welches überwiegend im Ueberschuss zugegeben wird, auch in Form eines Ammoniak abgebenden Mittels eingesetzt werden, wobei die Freisetzung bei erhöhter Temperatur und gegebenenfalls unter Druck erfolgt. Als Ammoniak abgebende Mittel kommen z.B. Ammoniumsalze von Niederalkancarbonsäuren, vorzugsweise Ammoniumacetat, oder einer Carbonsäure der Formel $R_3$-A-COOH, ferner ein geeignetes Niederalkancarbonsäureamid, insbesondere Formamid, in Frage.

Eine erfindungsgemäss erhältliche Verbindung kann in üblicher Weise in eine andere Verbindung der Formel I überführt werden.

Ein verfahrensgemäss erhältliches Acetal der Formel I ($R_3'$ = acetalisiertes Formyl) kann in üblicher Weise, erforderlichenfalls in Gegenwart eines geeigneten Hydrolysemittels zu einem Aldehyd der Formel I ($R_3'$ = gegebenenfalls hydratisiertes Formyl) hydrolysiert werden. Geeignete Hydrolysemittel sind beispielsweise Protonensäuren, wie Mineralsäuren, z.B. Chlor- oder Bromwasserstoffsäure, Schwefelsäure oder Phosphorsäure, oder organische Carbon- oder Sulfonsäuren, wie Niederalkansäuren, z.B. Essigsäure, oder Niederalkyl- bzw. Arylsulfonsäure, z.B. p-Toluolsulfonsäure. Umgekehrt kann man einen verfahrensgemäss erhältlichen Aldehyd (I, $R_3'$ = Formyl) in üblicher Weise, z.B. durch Umsetzung mit dem entsprechenden Alkohol in Gegenwart eines sauren Kondensationsmittels, wie einer Mineralsäure oder organischen Sulfonsäure, vorteilhaft unter destillativer bzw. azeotrop-destillativer Entfernung des Reaktionswassers, oder durch Umsetzung mit einem Orthocarbonsäureester, z.B. einem Orthoameisensäureniederalkylester, oder mit einem Acetal, bzw. Ketal einen z.B. mit Benzaldehyddiniederalkylacetal bzw. -niederalkylenacetal bzw. einem Acetophenon-diniederalkylketal bzw.-niederalkylenketal, vorteilhaft in Gegenwart katalytischer Mengen einer Mineral- oder Sulfonsäure, z.B. von Chlorwasserstoff- oder p-Toluolsulfonsäure, in Acetale (I, $R_3'$ = acetalisiertes Formyl) überführen.

Enthält mindestens einer der Substituenten $R_1$, $R_2$ und $R_3$ als zusätzlichen Substituenten Hydroxy, so lässt sich dieser nach an sich bekannter Weise verethern. Die Umsetzung mit einer Alkoholkomponente,

z.B. mit einem Niederalkanol, wie Ethanol, in Gegenwart von Säuren, z.B. Mineralsäure, wie Schwefelsäure, oder von Dehydratisierungsmitteln, wie Dicyclohexylcarbodiimid, führt zu Niederalkoxy.Phenole bzw. deren Salze lassen sich z.B. in Gegenwart von Basen, wie Alkalimetallhydroxiden oder -carbonaten, z.B. Natriumhydroxid oder Kaliumcarbonat, mit Hilfe von Diniederalkylsulfaten, Diazoniederalkanen oder Alkyl- bzw. Arylhalogeniden in entsprechende Niederalkylphenylether bzw. Arylphenylether überführen. Umgekehrt kann man Ether in Alkohole spalten. So entstehen z.B. aus Alkoxyarylverbindungen aromatische Alkohole, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, oder mittels Basen, z.B. Niederalkylaminen, wie Methylamin, durchführt.

Weiterhin lässt sich Hydroxy in Niederalkanoyloxy umwandeln, beispielsweise durch Umsetzung mit einer gewünschten Niederalkancarbonsäure, wie Essigsäure oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels. Umgekehrt kann verestertes Hydroxy, z.B. durch Basen-Katalyse, zu Hydroxy solvolysiert werden.

Erhaltene freie Verbindungen der Formel I können in an sich bekannter Weise in Salze überführt werden. Hydroxy aufweisende Gruppen $R_1$ bzw. $R_2$ werden mit entsprechenden Basen wie Alkalimetallhydroxiden, in die eingangs aufgeführten Salze mit Basen, oder durch Behandeln mit einer wie vorstehend aufgeführten, Säureadditionssalze bildenden Säureadditionssalze umgewandelt werden.

Erhaltene Salze können in an sich bekannter Weise in die freien Verbindungen umgewandelt werden, z.B. durch Behandeln mit einem sauren Reagenz, wie einer Mineralsäure, bzw. einer Base, z.B. Alkalihydroxid.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die Verbindungen der Formel (I) kann, je nach der Wahl der Ausgangsstoffe und Arbeitsweisen, in Form eines der möglichen Isomeren oder als Gemische derselben vorliegen.

Die Verbindung der Formel (I) einschliesslich ihre Salze kann auch in Form ihrer Hydrate erhalten werden oder andere zur Kristallisation verwendete Lösungsmittel einschliessen.

Die Verbindungen der Formel (I) können, je nach der Wahl der Ausgangsstoffe und Arbeitsweisen, in Form eines der möglichen Isomeren oder als Gemische derselben, z.B. je nach der Anzahl der asymmetrischen Kohlenstoffatome als reine optische Isomere, wie Antipoden, oder als Isomerengemische, wie Racemate, Diastereoisomerengemische oder Racematgemische, ferner als Tautomere vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung

eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Die Ausgangsstoffe der Formeln II, IV und V, die speziell für die Herstellung der erfindungsgemässen Verbindungen entwickelt wurden, die Verfahren zu ihrer Herstellung sowie ihre Verwendung bilden ebenfalls einen Gegenstand der Erfindung.

Bei den erfindungsgemässen pharmazeutischen Präparaten, welche die erfindungsgemässe Verbindung oder pharmazeutisch verwendbaren Salze davon enthalten, handelt es sich vorzugsweise um solche zur topischen Anwendung an Warmbrüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applicationsweise ab. Entsprechende Mittel mit einem Konzentrationsbereich von etwa 1 bis etwa 10 % G/G, z.B. in Form von Cremen, Salben oder Lösungen, können beispielsweise 2 bis 3 x täglich appliziert werden.

Als topisch anwendbare pharmazeutische Präparate kommen in erster Linie Cremen, Salben, Pasten, Schäume, Tinkturen und Lösungen in Frage, die von etwa 0,1 bis etwa 10 % des Wirkstoffs enthalten.

Cremen sind Oel-in-Wasser Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfessäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine,

ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele illustrieren die oben beschriebene Erfindung, sie sollen jedoch diese in ihrem Umfang in keiner Weise einschränken. Temperaturen sind in Celsiusgraden angegeben.

Beispiel 1:

Eine Mischung von 30,0 g α-Brom-benzyl-(3-pyridyl)-keton-hydrobromid und 50,0 g Ammoniumsalz des Malonaldehydsäure-dimethylacetals in 180 ml wasserfreiem Dimethylformamid werden unter Rühren und Einleiten von Stickstoffgas während vier Stunden auf 100° erhitzt. Dann wird die Mischung abgekühlt und bei 70° unter 11 Torr zur Trockene eingedampft. Den Rückstand versetzt man mit 100 ml Wasser und 400 ml Ethylacetat. Durch Zugabe von konzentrierter wässriger Ammoniaklösung wird der pH auf 8,0 gestellt. Die organische Phase wird abgetrennt, mit 50 ml Wasser gewaschen, über Magnesiumsulfat getrocknet und unter 11 Torr zur Trockene eingedampft. Den Rückstand chromatographiert man an 500 g Silikagel. Die Fraktionen 1-4, eluiert mit je 600 ml Chloroform, werden verworfen. Die Fraktionen 5-16, eluiert mit je 600 ml Chloroform-Methanol (98:2), werden vereinigt und unter vermindertem Druck zur Trockene eingedampft. Der Rückstand, das N-[α-(3-Pyridyl)-phenacyl]-malonaldehyd-säure-dimethylacetal-amid liegt als gelbes Oel vor.

Die Fraktionen 19-24, eluiert mit je 600 ml Chloroform-Methanol (97:3), werden vereinigt und unter vermindertem Druck zur Trockene eingedampft. Der Rückstand wird auf Ether-Petrolether kristallisiert.

Das 2-[4-(5)-Phenyl-5(4)-(3-pyridyl)-imidazol-2-yl]-acetaldehyd-dimethylacetal schmilzt bei 142-145°.

Das Ausgangsmaterial kann wie folgt hergestellt werden:
Ein Gemisch von 10,0 g N-[α-(3-Pyridyl)-phenacyl]-malonaldehyd-säure-dimethylacetal-amid, 30,0 g Ammoniumacetat und 100 ml Eisessig wird zwei Stunden unter Rückfluss gekocht und anschliessend unter kräftigem Rühren in ein Gemisch aus 200 g Eis und 150 ml konzentrierter wässriger Ammoniaklösung gegossen. Der Kristallbrei wird zweimal mit je 150 ml Ethylacetat extrahiert und die organische Phase mit Wasser neutral gewaschen, mit Magnesiumsulfat getrocknet und unter 11 Torr bei 40° zur Trockene eingedampft. Den Rückstand chromatographiert man an 500 g Silikagel. Die Fraktionen 1-4, eluiert mit je 500 ml Chloroform-Methanol (99:1), werden verworfen. Die Fraktionen 5-15, eluiert mit je 500 ml Chloroform-Methanol (99:2), werden vereinigt und unter vermindertem Druck zur Trockene eingedampft. Den Rückstand kristallisiert man aus Ether-Petrolether. Das 2-[4(5)-Phenyl-5(4)-(3-pyridyl)-imidazol-2-yl]-acetaldehyd-dimethylacetal schmilzt bei 142-145°.

Der Ausgangsstoff wird wie folgt hergestellt: 19,7 g Malonaldehyd-säure-dimethylacetal (V.V. Shikina et al. J. Gen. Chem. U.S.S.R. 25, 723-725 (1955)) werden in 300 ml wasserfreiem Ether gelöst. In die Lösung wird bei 0° während einer Stunde Ammoniakgas eingeleitet. Anschliessend wird die Mischung unter vermindertem Druck zur Trockene eingedampft. Das Ammoniumsalz des Malonaldehyd-säure-dimethylacetals liegt als Oel vor.

Beispiel 2: Eine Lösung von 5,9 g 2-[4(5)-Phenyl-5(4)-(3-pyridyl)-imidazol-2-yl]-acetaldehyd-dimethylacetal in 120 ml Methylenchlorid wird auf 0-5° abgekühlt und mit 3,5 g m-Chlorperbenzoesäure versetzt.Die Mischung wird 24 Stunden bei Raumtemperatur gerührt.Die gelbe Lösung wird anschliessend zweimal mit je 20 ml 2 N Kaliumhydrogenkarbonatlösung und mit 30 ml Wasser gewaschen, über Magnesiumsulfat getrocknet und bei 40° unter vermindertem Druck eingeengt. Der Rückstand wird in wenig Methanol gelöst. Nach Zusatz von Wasser kristallisiert der 2-[4(5)-Phenyl-5(4)-(1-oxido-3-pyridyl)-imidazol-2-yl]-acetaldehyd-dimethylacetal.

Beispiel 3: Eine Suspension von 3,29 g 2-[4(5)-Phenyl-5(4)-(3-pyridyl)-imidazol-2-yl]-2-methyl-propionsäure-Natriumsalz in 70 ml wasserfreiem Benzol wird unter Rühren während 10 Minuten bei 5° 3,2 ml Oxalylchlorid zugetropft. Die Mischung wird eine Stunde bei 5° und 15 Stunden bei Raumtemperatur gerührt, abgekühlt und unter vermindertem Druck zur Trockene eingeengt. Den Rückstand versetzt man mit 70 ml wasserfreiem Benzol und engt wieder unter vermindertem Druck zur Trockene ein. Das 2-[4(5)-Phenyl-5(4)-(3-pyridyl)-imidazol-2-yl]-2-methyl-propionsäurechlorid liegt als Oel vor. Das Säurechlorid ist unstabil und wird sofort umgesetzt.

3,4 g g frisch hergestelltes 2-[4(5)-Phenyl-8(4)-(3-pyridyl)-imidazol-2-yl]-2-methyl-propionsäurechlorid werden in 60 ml Diethylenglykoldimethylester gelöst. Die Lösung wird auf -75° abgekühlt und unter Rühren und Einleiten von Stickstoffgas während 30 Minuten mit einer Suspension von 2,54 g Lithium-tri-tert.butoxy-aluminiumhydrid versetzt. Die Suspension wird nach beendetem Zutropfen noch 30 Minuten bei -70° und eine Stunde ohne Kühlung gerührt, wobei die Innentemperatur auf 0° ansteigt. Man giesst die Mischung auf 500 ml Eiswasser und filtriert durch eine Schicht Hyflo Super Cd. Das Filtrat wird mit konzentrierter wässriger Ammoniaklösung auf pH 8,0 gestellt. Das Gemisch wird mit 100 ml Methylenchlorid geschüttelt. Man filtriert die Wasser-Methylenchloridmischung durch eine Schicht Hyflo Super Cd. Das Filtrat wird dreimal mit je 40 ml Methylenchlorid extrahiert. Man vereinigt die organischen Extrakte, wäscht mit 50 ml Wasser und engt unter vermindertem Druck zur Trockene ein. Anschliessend wird bei 50° unter 0,1 mm vom Diethylenglykoldimethylether befreit. Der Rückstand chromatographiert man an einer Lobar Fertigsäule (Grösse C, Merck) für Niederdruck-Flüssigkeits-Chromatographie. Die Fraktionen 1-14, eluiert mit

je 10 ml Chloroform-Isopropanol (95:5), werden verworfen. Die Fraktionen 15-29, eluiert mit je 10 ml Chloroform-Isopropanol (85:15) werden vereinigt und unter vermindertem Druck zur Trockene eingedampft. Der Rückstand, der 2-[4(5)-Phenyl-5(4)-(3-pyridyl)-imidazol-2-yl]-2-methylpropionaldehyd liegt als Oel vor.

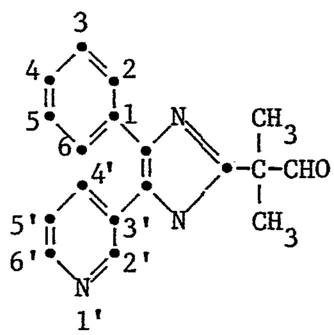

NMR ($CDCl_3$): 9,70 (s, CHO), 8,68 (br, H-2'), 8,34 (dbr, H-6'), 7,85 (dm, H-4'), 7,1-7,5 (m, übrige arom. H), 1,60 (s, $CH_3$).

Beispiel 4: In analoger Weise wie in Beispiel 1-3 beschrieben sowie entsprechend der in der Beschreibung aufgezeigten Weise kann man erhalten:

2-[4(5)-Phenyl-5(4)-(3-pyridyl)-imidazol-2-yl]-propanoldimethylacetal, Oel, NMR ($CDCl_3$), 8,72 (br. H-2'), 8,45 (dbr, H-6'), 7,84 (dm, H-4'), 7,1-7,5 (m, übrige Arom. H), 4,61 (d, CH-O), 3,4 ($OCH_3$), 3,51 (dq, $C\underline{H}-CH_3$), 1,63 (d, $C-C\underline{H}_3$);

2-[4(5)-Phenyl-5(4)-(3-pyridyl)-imidazol-2-yl]-2-methyl-propanol-dimethylacetal, Oel, NMR ($CDCl_3$): 8,73 (br, H-2'), 8,41 (dbr, H-6'), 7,85 (dm, H-4'), 7,1-7,5 (m, übrige Arom. H), 4,60 (s, CH), 3,47 (s, $OCH_3$), 1,65 ($CC\underline{H}_3$ );

2-[4(5)-Phenyl-5(4)-(1-oxido-3-pyridyl)-imidazol-2-yl]-propanol-dimethylacetol, Oel;

2-[4(5)-Phenyl-5(4)-(1-xoido-3-pyridyl)-imidazol-2-yl]-2-methyl-propanol-dimethylacetol, Oel;

2-[4(5)-Phenyl-5(4)-(3-pyridyl)-imidazol-2-yl]-2-methyl-1,1-methylendioxypropan, Oel;

2-[4(5)-Phenyl-5(4)-(3-pyridyl)-imidazol-2-yl]-2-methyl-1,1-ethylendioxypropan, Oel;

2-[4(5)-Phenyl-5(4)-(3-pyridyl)-imidazol-2-yl]-butyraldehyd, Oel;

2-[4(5)-Phenyl-5(4)-(3-pyridyl)-imidazol-2-yl]-acetaldehyd, Oel, NMR ($CDCl_3$): 9,70 (s, CHO), 8,71 (br, H-2'), 8,35 (dbr, H-6'), 7,84 (dm, H-4'), 7,1-7,5 (m, übrige Arom. H), 4,03 (s, $CH_2$);

2-[4(5)-Phenyl-5(4)-(3-pyridyl)-imidazol-2-yl]-propionaldehyd, Oel, NMR ($CDCl_3$): 9,72 (s, CHO), 8,73 (br, H-2'), 8,40 (br, H-6'), 7,85 (dbr, H-4'), 7,1-75, (m, übrige Arom. H), 3,97 (q, C$\underline{H}CH_3$), 1,65 (d, $CH_3$);

2-[4(5)-Phenyl-5(4)-(1-oxido-3-pyridyl)-imidazol-2-yl]-acetaldehyd, Oel;

2-[4(5)-Phenyl-5(4)-(1-oxido-3-pyridyl)-imidazol-2-yl]-propionaldehyd, Oel;

2-[4(5)-Phenyl-5(4)-(1-oxido-3-pyridyl)-imidazol-2-yl]-2-methyl-propionaldehyd, Oel;

2-[4(5)-Phenyl-5(4)-(1-oxido-3-pyridyl)-imidazol-2-yl]-butyraldehyd, Oel, ausgehend aus dem entsprechenden Natriumsalz der jeweiligen Säure über das jeweilige Säurechlorid.

Beispiel 5: Eine Salbe, enthaltend 5 % 2-[4(5)-Phenyl-5(4)-(1-oxido-3-pyridyl)-imidazol-2-yl]-2-methylpropanal-dimethylacetal, kann wie folgt hergestellt werden:

Zusammensetzung:

| | |
|---|---|
| Wirkstoff | 5,0 % |
| Vaseline | 45,0 % |
| Paraffinöl | 19,6 % |
| Cetylalkohol | 5,0 % |
| Bienenwachs | 5,0 % |
| Sorbitan-sesquioleat | 5,0 % |
| p-Hydroxybenzoesäureester | 0,2 % |
| Wasser , entmineralisiert bis zu | 100,0 % |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel 6: Eine Crème, enthaltend 10 % 2-[4(5)-Phenyl-5(4)-(3-pyridyl)-imidazol-2-yl]-acetaldehyd-dimethylacetal, kann wie folgt hergestellt werden:

Zusammensetzung:

| | |
|---|---|
| Wirkstoff | 10,0 % |
| Isopropylpalmitat | 8,0 % |
| Cetylpalmitat | 1,5 % |
| Siliconöl 100 | 0,5 % |
| Sorbitanmonostearat | 3,0 % |
| Polysorbat 60 | 3,5 % |
| 1,2-Propylenglykol PH | 20,0 % |
| Acrylsäurepolymerisat | 0,5 % |
| Triethanolamin | 0,7 % |
| Wasser, entmineralisiert bis zu | 100,0 % |

Das Acrylsäurepolymerisat wird in einem Gemisch aus entmineralisiertem Wasser und 1,2-Propylenglykol suspendiert. Unter Rühren wird hierauf Triethanolamin zugegeben. wodurch ein Schleim erhalten wird. Ein Gemisch aus Isopropylpalmitat, Cetylpalmitat, Siliconöl, Sorbitanmonostearat und Polysorbat wird auf ca. 75° erwärmt und unter Rühren in den gleichfalls auf ca. 75° erwärmten Schleim eingearbeitet. Die auf Raumtemperatur abgekühlten Crème-Grundlage wird hierauf zur Herstellung eines Konzentrates mit dem Wirkstoff verwendet. Das Konzentrat wird mittels eines Durchlaufhomogenisators homogenisiert und dann protionsweise der Grundlage hinzugefügt.

Beispiel 7: Eine Crème, enthaltend 5 % 2-[4(5)-Phenyl-5(4)-(1-oxido-3-pyridyl)-imidazol-2-yl]-2-methylpropanal-dimethylacetal, kann wie folgt erhalten werden.

Zusammensetzung:

| | |
|---|---|
| Wirkstoff | 5,0 % |
| Cetylpalmitat PH | 2,0 % |
| Cetylalkohol PH | 2,0 % |
| Triglyceridgemisch gesättigter mittelfettiger Fettsäuren | 5,0 % |
| Stearinsäure | 3,0 % |
| Glycerinstearat PH | 4,0 % |
| Cetomacrogol 1000 | 1,0 % |
| mikrokristalline Cellulose | 0,5 % |
| 1,2-Propylenglykol, dest. | 20,0 % |
| Wasser, entmineralisiert, bis zu | 100,0 % |

Cetylalkohol, Cetylpalmitat, das Triglyceridgemisch, Stearinsäure und Glycerinstearat werden zusammengeschmolzen. Die mikrokristalline Cellulose wird in einen Teil des Wassers dispergiert. Im restlichen Teil des Wassers wird Cetomacrogol gelöst und das Propylenglykol sowie der Schleim dazu gemischt. Die Fettphase wird anschliessend unter Rühren zu Wasserphase gegeben und kaltgerührt. Schliesslich wird der Wirkstoff mit einem Teil der Grundlage angerieben und hierauf die Anreibung in den Rest der Crème eingearbeitet.

Beispiel 8: Ein transparentes Hydrogel, enthaltend 5 % 2-(4(5)-Phenyl-5(4)-(4-pyridyl)-imidazol-2-yl]-acetaldehyd-dimethylacetal, wird wie folgt hergestellt.

Zusammensetzung:

| | |
|---|---|
| Wirkstoff | 5 % |
| Propylenglykol | 10 - 20 % |
| Isopropanol | 20 % |
| Hydroxypropyl-methylcellulose | 2 % |
| Wasser | ad 100 % |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit einem gequollenen Cellulose-Derivat vermischt und, wenn erwünscht, mit Riechstoffen (0,1 %) versetzt.

Beispiel 9: Ein transparentes Hydrogel, enthaltend 5 % 2-[4(5)-Phenyl-5(4)-(1-oxido-3-pyridyl)-imidazol-2-yl]-2-methylpropanal-dimethylacetal, wird wie folgt hergestellt.

Zusammensetzung:

| | |
|---|---|
| Wirkstoff | 5 % |
| Propylenglykol | 20 % |
| Isopropanol | 20 % |
| Acrylsäurepolymerisat | 2 % |
| Triethanolamin | 3 % |
| Wasser | ad 100 % |

Acrylsäurepolymerisat und Wasser werden dispergiert und mit Triethanolamin neutralisiert. Der Wirkstoff wird in einem Gemisch aus Isopropanol und Propylenglykol gelöst. Anschliessend wird die Wirkstofflösung mit dem Gel vermischt, wobei, wenn erwünscht, Riechstoff (0,1 %) zugegeben werden kann.

Beispiel 10: Ein Schaumspray, enthaltend 1 % 2-[4(5)-Phenyl-5(4)-(1-oxido-3-pyridyl)-imidazol-2-yl]-2-methylpropanal-dimethylacetal, kann folgendermassen hergestellt werden:

Zusammensetzung:

| | |
|---|---|
| Wirkstoff | 1,00 % |
| Cetylalkohol PH | 1,70 % |
| Paraffinöl, dickflüssig | 1,00 % |
| Isopropylmyristat | 2,00 % |
| Cetomacrogol 1000 | 2,40 % |
| Sorbitanmonostearat | 1,50 % |
| 1,2-Propylenglykol PH | 5,00 % |
| Methylparaben | 0,18 % |
| Propylparaben | 0,02 % |
| Chemoderm 314 | 0,10 % |
| Wasser, entmineralisiert, bis zu | 100,00 % |

Cetylalkohol, Paraffinöl, Isopropylmyristat, Cetomacrogol und Sorbitanstearat werden zusammengeschmolzen. Methyl- und Propylparaben werden in heissem Wasser gelöst. Die Schmelze und die Lösung werden anschliessend vermischt. Der Wirkstoff, in Propylenglykol suspendiert, wird in die Grundlage eingearbeitet. Anschliessend wird Chemoderm zugeführt und mit Wasser auf das Endgewicht ergänzt.

Abfüllung:

20 ml des Gemisches wird in eine Aluminiumblockdose eingefüllt. Die Dose wird mit einem Ventil versehen, und das Treibgas wird unter Druck eingefüllt.

Patentansprüche

1. Neue substituierte Diazaverbindungen der allgemeinen Formel

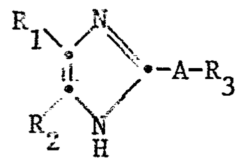

(I),

worin $R_1$ und $R_2$ unabhängig voneinander carbocyclisches Aryl und/oder Heteroaryl bedeuten, A einen zweiwertigen Kohlenwasserstoffrest darstellt und $R_3$ Formyl oder acetalisiertes Formyl bedeutet, ihre Isomeren und ihre Salze.

2. Verbindungen der Formel (I) gemäss Anspruch 1, worin einerseits $R_1$ und $R_2$ unabhängig voneinander Phenyl und/oder durch Halogen, Niederalkyl, Hydroxy, Niederalkoxy und/oder Niederalkanoyl substituiertes Phenyl bedeuten oder worin andererseits einer der Reste $R_1$ und $R_2$ Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Phenyl, Pyrrolyl, Furyl, Thienyl, Pyridyl, 1-Oxidopyridyl oder Pyrimidyl bedeutet, welches jeweils unsubstituiert oder durch Halogen, Niederalkyl, Hydroxy, Niederalkoxy und/oder Niederalkanoyloxy substituiert sein können, und jeweils A Niederalkylen, Niederalkyliden, Niederalkenylen, Niederalkenyliden, Cycloalkylen, Cycloalkyliden oder Cycloalkyl-niederalkyliden darstellen und $R_3$ Formyl oder mit einem aliphatischen Alkohol acetalisiertes Formyl bedeutet, ihre Isomeren sowie ihre Salze.

3. Verbindungen der Formel (I) gemäss Anspruch 1, worin $R_1$ und $R_2$ unabhängig voneinander Phenyl und/oder Halogen, Hydroxy, Niederalkyl, Niederalkoxy und/oder Niederalkanoyloxy substituiertes Phenyl bedeutet, A Niederalkylen mit bis und mit 4 C-Atomen, wie Methylen, Niederalkyliden mit bis und mit 7 C-Atomen, wie 2,2-Propyliden, Niederalkenylen mit bis und mit 4 C-Atomen, wie 1,3-Propen-2-ylen, Niederalkenyliden mit bis und mit 7 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkylniederalkyliden mit bis und mit 7 C-Atomen im Alkylidenteil und mit einem 3- bis 8-gliedrigen Cycloalkylteil, wie 2-Cyclohexyl-1,1-ethyliden, bedeutet und $R_3$ Formyl oder mit einem Niederalkanol, Niederalkenol, Niederalkandiol oder Niederalkendiol acetalisiertes Formyl bedeutet, ihre Isomeren sowie ihre Salze.

4. Verbindungen der Formel (I) gemäss Anspruch 1, worin einer der Reste $R_1$ und $R_2$ Pyridyl oder 1-Oxido-pyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, wie Methylen, Niederalkyliden mit bis und mit 7 C Atomen, wie 2,2-Propyliden, Niederalkenylen mit bis und mit 7 C-Atomen, wie 1,3-Propen-2-ylen, Niederalkenyliden mit bis und mit 4 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkyl-niederalkyliden mit bis und mit 7 C-Atomen im Alkylidenteil und mit einem 3- bis 8-gliedrigem Cycloalkylteil, wie 2-Cyclohexyl-1,1-ethyliden, bedeutet und $R_3$ Formyl oder mit einem Niederalkanol, Niederalkenyl, Niederalkandiol oder Niederalkendiol acetalisiertes Formyl bedeutet, ihre Isomeren sowie ihre Salze.

5. Verbindungen der Formel (I) gemäss Anspruch 1, worin $R_1$ und $R_2$ unabhängig voneinander Phenyl und/oder durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeuten, A Niederalkylen mit bis und mit 4 C-Atomen, wie Methylen, Niederalkyliden mit bis und mit 7 C-Atomen, wie 2,2-Propyliden, Niederalkenyliden mit bis und

mit 7 C-Atomen, wie 1,1-Buten-3-yliden, oder 3- bis 8-gliedriges Cycloniederalkyliden, wie 1,1-Cyclopentyliden, darstellen und $R_3$ Formyl oder Diniederalkoxy-methyl jeweils mit bis und mit 4 C-Atomen im Niederalkoxyteil, wie Dimethoxy-methyl, oder Niederalkendioxy-methyl mit bis und mit 4 C-Atomen im Niederalkylenteil, wie 1,3-Dioxolan-2-yl, bedeutet, ihre Isomeren sowie ihre Salze.

6. Verbindungen der Formel (I) gemäss Anspruch 1, worin einer der Reste $R_1$ und $R_2$ Phenyl oder durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeutet und der andere Pyridyl, wie 3-Pyridyl, oder 1-Oxido-pyridyl, wie 1-Oxido-3-pyridyl, bedeutet, die jeweils unsubstituiert oder durch Halogen mit Atomnummer bis und mit 35, wie Chlor, Hydroxy und/oder Niederalkoxy mit bis und mit 4 C-Atomen, wie Methoxy, substituiert sein können, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, darstellen und $R_3$ Formyl oder Diniederalkoxymethyl, jeweils mit bis und mit 4 C-Atomen im Niederalkylteil, wie Dimethoxymethyl, oder Niederalkylendioxy-methyl mit bis und mit 4 C-Atomen im Niederalkylenteil, wie 1,3-Dioxolan-2-yl, bedeutet, ihre Isomeren sowie ihre Salze.

7. Verbindungen der Formel (I) gemäss Anspruch 1, worin $R_1$ und $R_2$ unabhängig voneinander Phenyl und/oder durch Niederalkoxy mit bis und mit 4 C-Atomen, wie Methoxy, substituiertes Phenyl bedeuten, A Niederalkylen mit bis und mit 4 C-Atomen, wie Methylen, oder insbesondere Niederalkyliden mit bis und mit 4 C-Atomen, wie 2,2-Propyliden, darstellt und $R_3$ Diniederalkoxy-methyl jeweils mit bis und mit 4 C-Atomen im Niederalkoxyteil, wie Diethoxy-methyl, oder Niederalkylendioxy-methyl mit bis und mit 4 C-Atomen im Niederalkylenteil, wie 1,3-Dioxolan-2-yl, bedeutet, ihre Isomeren sowie ihre Salze.

8. Verbindungen der Formel (I) gemäss Anspruch 1, worin einer der Reste $R_1$ und $R_2$ Phenyl oder durch Halogen mit Atomnummer bis und mit 35, wie Chlor, Hydroxy oder Niederalkoxy mit bis und mit 4 C-Atomen, wie Methoxy, substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A Niederalkyliden mit bis und mit 4 C-Atomen, wie 2,2-Propyliden, bedeutet, und $R_3$ Formyl oder Diniederalkoxy-methyl jeweils mit bis und mit 4 C-Atomen im Niederalkoxyteil, wie Diethoxy-methyl, oder Niederalkylendioxy-methyl mit bis und mit 4 C-Atomen im Niederalkylenteil, wie 1,3-Dioxolan-2-yl, bedeutet, ihre Isomeren sowie ihre Salze.

9. Verbindungen der Formel (I) gemäss Anspruch 1, worin einer der Reste $R_1$ und $R_2$ Phenyl oder durch Halogen mit Atomnummer bis und mit 35, wie Chlor, Hydroxy oder Niederalkoxy mit bis und mit 4 C-Atomen, wie Methoxy,substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A ein ein quartäres C-Atom aufweisendes Niederalkyliden mit bis und mit 4 C-Atomen, wie 2,2-Propyliden, wobei das quartäre C-Atom direkt an den Imidazolring gebunden ist, bedeutet, und $R_3$ Formyl oder Diniederalkoxy-methyl mit bis und mit 4 C-Atomen jeweils im Niederalkylteil, wie Diethoxy-methyl, darstellt, ihre Isomeren sowie ihre Salze.

10. Verbindungen der Formel (I) gemäss Anspruch 1, worin einer der Reste $R_1$ und $R_2$ Phenyl und der andere Pyridyl, wie 3-Pyridyl, oder 1-Oxidopyridyl, wie 1-Oxido-3-pyridyl, bedeutet, A 2,2-Propyliden darstellt und $R_3$ Formyl oder Diniederalkoxymethyl jeweils mit bis und mit 4 C-Atomen im Niederalkoxy, wie Diethoxymethyl, bedeutet, ihrer Isomeren sowie ihre Salze.

11. 2-[4(5)-Phenyl-5(4)-(3-pyridyl)-imidazol-2-yl]-acetaldehyd-dimethylacetal.

12. 2-[4-(5)-Phenyl-5(4)-(1-oxido-3-pyridyl)-imidazol-2-yl]-acetaldehyd-dimethylacetyl.

13. 2-[4(5)-Phenyl-5-(4)-(3-pyridyl)-imidazol-2-yl]-propandimethylacetal.

14. 2-[4-(5)-Phenyl-5(4)-(3-pyridyl)-imidazol-2-yl]-methyl-propanaldimethylacetal.

15. 2-[4(5)-Phenyl-5(4)-(1-oxido-3-pyridyl)-imidazol-2-yl]-propanaldimethylacetal.

16. 2-[4(5)-Phenyl-5(4)-(1-oxido-3-pyridyl)-imidazol-2-yl]-2-methylpropanaldimethylacetal.

17. 2-[4(5)-Phenyl-5(4)-(3-pyridyl)-imidazol-2-yl]-2-methyl-1,1-methylendioxypropan.

18. 2-[4(5)-Phenyl-5(4)-(3-pyridyl)-imidazol-2-yl]-2-methyl-1,1-ethylendioxypropan.

19. 2-[4(5)-Phenyl-5(4)-(3-pyridyl)-imidazol-2-yl]-2-methyl-propionaldehyd.

20. 2-[4(5)-Phenyl-5(4)-(3-pyridyl)-imidazol-2-yl]-2-methyl-butyraldehyd.

21. 2-[4(5)-Phenyl-5(4)-(3-pyridyl)-imidazol-2-yl]-propionaldehyd.

22. 2-[4(5)-Phenyl-5(4)-(1-oxido-3-pyridyl)-imidazol-2-yl]-acetaldehyd.

23.2-(4(5)-Phenyl-5(4)-(1-oxido-3-pyridyl)-imidazol-2-yl]-propionaldehyd, 2-[4(5)-Phenyl-5(4)-(1-oxido-3-pyridyl)-imidazol-2-yl]-2-methyl-propionaldehyd, 2-[4(5)-Phenyl-5(4)-(1-oxido-3-pyridyl)-imidazol-2-yl]-butyraldehyd.

24. Eine Verbindung gemäss einem der Ansprüche 11 bis 23 in Form eines Säureadditionssalzes.

25. Verbindung gemäss einem der Ansprüche 1-24 mit Wirkung als externe Hautphlogistika.

26. Pharmazeutische Präparate, enthaltend mindestens eine Verbindung gemäss einem der Ansprüche 1-25.

27. Verfahren zur Herstellung von Verbindungen der Formel (I) gemäss Anspruch 1, dadurch gekennzeichnet, dass man aus einer Verbindung der Formel

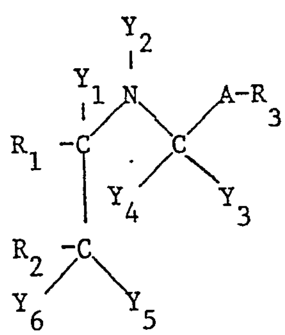

(II) ,

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N- bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel -NH- darstellt oder worin $Y_1$ gemeinsam mit $Y_6$ eine Bindung darstellt, $Y_2$ gemeinsam mit $Y_3$ eine zusätzliche Bindung oder reaktionsfähiges verestertes Hydroxy, insbesondere Halogen oder Sulfonyloxy, darstellt oder worin $Y_1$ Hydroxy

ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo oder Imino bedeutet, oder einem Tautomeren und/oder Salz davon, unter Einführung einer gegebenenfalls zusätzlichen Bindung H-Z abspaltet, oder eine Verbindung der Formel

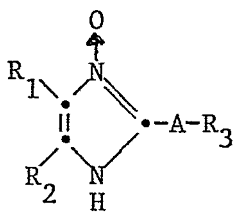 (IV)

oder ein Salz davon zu einer Verbindung der Formel I reduziert, in einer Verbindung der Formel

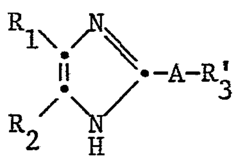

worin $R_3'$ einen in $R_3$ überführbaren Rest bedeutet, oder in einem Salz davon $R_3'$ in $R_3$ überführt und gewünschtenfalls die verfahrensgemäss erhältliche Verbindung in eine andere Verbindung der Formel I, eine verfahrensgemäss erhältliche Verbindung in ein Salz oder ein verfahrensgemäss erhältliches Salz in die freie Verbindung oder in ein anderes Salz überführt und/ oder, wenn erwünscht, ein erfindungsgemäss erhältliches Gemisch von isomeren Verbindungen der Formel I in die einzelnen Isomeren auftrennt.

28. Verfahren gemäss Anspruch 27, dadurch gekennzeichnet, dass man von einer auf irgendeiner Stufe des Verfahrens als Zwischenprodukt erhältlichen Verbindung ausgeht und die fehlenden Schritte durchführt. oder einen Ausgangsstoff in Form eines Salzes, Isomeren und/oder Racemates bzw. Antipoden verwendet oder unter den Reaktionsbedingungen bildet.

29. Verfahren zur Herstellung pharmazeutischer Präparate, dadurch

gekennzeichnet, dass man mindestens eine Verbindung gemäss einem der Ansprüche 1-25 mit üblichen pharmazeutischen Hilfs- und Trägerstoffen vermischt.

30. Verbindung gemäss einem der Ansprüche 1-25 zur therapeutischen Behandlung des menschlichen oder tierischen Körpers.

31. Verwendung von Verbindung gemäss einem der Ansprüche 1-25 in einem Verfahren zur Behandlung entzündlicher Dermatosen.

32. Die in den Beispielen 1-4 genannten neuen Verbindungen.

33. Das Verfahren der Beispiele 1-10 und die danach erhältlichen Verfahrensprodukte.

34. Die in dem Verfahren gemäss Anspruch 27, 28 und 32 verwendeten neuen Ausgangsstoffe und Zwischenprodukte.

FO 7.3/DH/rö*

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0084757

Nummer der Anmeldung

EP 82 81 0032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | CH - A - 561 718 (CIBA-GEIGY) | | C 07 D 233/64<br>401/04<br>403/04<br>405/04<br>409/04<br>A 61 K 31/44<br>31/33 |
| A | EP - A - 0 032 113 (DU PONT) | | |
| | ----- | | |

RECHERCHIERTE SACHGEBIETE (Int. Cl. 3)

C 07 D 233/00
401/00
403/00
405/00
409/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-26, 28 - 30

Unvollständig recherchierte Patentansprüche:

Nicht recherchierte Patentansprüche: 1)27, 2) 31, 3) 32,33 und 34(teilweise)

Grund für die Beschränkung der Recherche:

1) Anspruch 27 is unverstandlich (Siehe Zeile 10 der Beschreibung)
2) (Art.52(4) des Europäischen Patentübereinkommens) und (Regel 39.1 (IV)
3. (Regel 29.6, Regel 62a )

| Recherchenort | Abschlußdatum der Recherche | Prüfer |
|---|---|---|
| Den Haag | 24-09-1982 | DE BUYSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches
Patentamt

0084757

## ☐ GEBÜHRENPFLICHTIGE PATENTANSPRÜCHE

Die vorliegende europäische Patentanmeldung enthielt bei ihrer Einreichung mehr als zehn Patentansprüche.

☐ Alle Anspruchsgebühren wurden innerhalb der vorgeschriebenen Frist entrichtet. Der vorliegende europäische Recherchenbericht wurde für alle Patentansprüche erstellt.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt für die Anspruchsgebühren entrichtet wurden,

nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## ☒ MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung; sie enthält mehrere Erfindungen oder Gruppen von Erfindungen, nämlich:

1. Patentansprüche 1-31,33,32 (Teilweise) Imidazole der Formel I, Herstellung und Zusammensetzungen.
2. Patentansprüche 34,32 (Teilweise) Zwischenprodukte per se.

☐ Alle weiteren Recherchengebühren wurden innerhalb der gesetzten Frist entrichtet. Der vorliegende europäische Recherchenbericht wurde für alle Patentansprüche erstellt.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind,

nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen,

nämlich Patentansprüche: 1-31, 33, 32 (Teilweise)